# EUROPÄISCHE PATENTSCHRIFT

(11) **EP 1 020 192 B1**
(45) Veröffentlichungstag und Bekanntmachung des Hinweises auf die Patenterteilung: **19.06.2002**
(21) Anmeldenummer: 00250002.3
(22) Anmeldetag: 06.01.2000
(51) Int. Cl.: A61K 31/635, A61K 31/522, A61K 31/165, A61K 31/167, A61K 31/341, A61K 31/35, A61K 31/245, A61K 31/404, A61K 35/78, A61P 17/00

(54) **Anticellulitiszusammensetzung und ihre Verwendung**
Composition against cellulitis
Composition anti-cellulitique

(30) Priorität: 11.01.1999 DE 19901502
(43) Veröffentlichungstag der Anmeldung: 19.07.2000
(73) Patentinhaber: Schneider, Michael, 22453 Hamburg (DE)
(72) Erfinder: Schneider, Michael, 22453 Hamburg (DE); Wirsig, Dorle, 22523 Hamburg (DE)
(74) Vertreter: UEXKÜLL & STOLBERG

(56) Entgegenhaltungen:
- DE-A- 4 401 308
- US-A- 4 191 748
- MENAGUALE G. ET AL: "side effects of mesotherapie: report of three cases" DERMATOLOGICA CLINICA, Bd. 13, Nr. 3, 1993, Seiten 191-195, XP000917054
- GROSSHANS E.: "the treatment of cellulitis: useless or dangerous!" REVUE DU PRACTICIEN - MEDECINE GENERALE, Bd. 7, Nr. 214, 1993, Seiten 13-15, XP000917086
- BOMBARDELLI, E. ET AL: "Aesculus Hippocastanum L." FITOTERAPIA, Bd. 67, Nr. 6, 1996, Seiten 483-511, XP000917088

## Beschreibung

Die vorliegende Erfindung betrifft eine Zusammensetzung, deren Verwendung als Arzneimittel und kosmetisches Erzeugnis sowie ein Verfahren zur kosmetischen Behandlung von Cellulitis.

Unter Cellulitis versteht man im allgemeinen eine nichtentzündliche, konstitutionell bedingte umschriebene Degeneration der Kollagenen und elastischen Fasern des subkutanen Bindegewebes, besonders bei jüngeren Frauen in der Oberschenkel- und Glutäalregion. Zu dem symptomatischen Erscheinungsbild gehören das sogenannte Matratzenphänomen sowie die sogenannte Orangenschalenhaut. Prophylaktisch begegnet man der Cellulitis im allgemeinen durch Gewichtsreduktion und körperliches Training.

Dermatologia Clinica (1993) 13(3), Seiten 191-195 offenbart die Behandlung von Cellulitis mittels Mesotherapie mit Mitteln, die unter anderem die Wirkstoffe Theophyllin und Furosemid oder Troxerutin und Carbazochrom enthalten. Es wird offenbart, dass dabei Hautschädigungen wie Läsionen mit nachfolgender Entwicklung von Abzessen auftraten, die zu bleibenden Vernarbungen führten.

Die US-A-4 191 748 offenbart eine Zusammensetzung, die Theophyllin in Kombination mit einer fluorierten Verbindung und gegebenenfalls weitere wirksame Bestandteile enthält, zur Behandlung von Cellulitis.

Revue Du Practicien - Medecine Generale (1993) 7 (214), Seiten 13-15 beschreibt Risiken der Mesotherapie zur Behandlung von Cellulitis, wobei die Wirkstoffe Procain und Theophyllin als nicht lipolytisch wirksam beschrieben werden.

Die DE-A 44 01 308 offenbart ein kosmetisches Mittel für die Haut mit anticellulitischer und schlankmachender Wirkung, das neben einer Vielzahl anderer Bestandteile Rosskastanienextrakt enthält.

Fitoterapia (1996) 67(6), Seiten 483-511 ist eine Monographie über Rosskastanienextrakt sowie dessen Anwendungen in Medizin und Kosmetik, wobei auch die Behandlung von Cellulitis erwähnt wird.

Der vorliegenden Erfindung liegt somit die Aufgabe zugrunde, eine Zusammensetzung zu schaffen, die das symptomatische Erscheinungsbild der Cellulitis verringert und gegebenenfalls ganz beseitigt, lokal, d.h. im Bereich der betroffenen Hautbereiche, angewendet werden kann und zugleich einfach in der Handhabung ist sowie gegebenenfalls weitere medizinische Anwendungen erlaubt, wobei die im Stand der Technik beschriebenen kosmetisch äußerst nachteiligen und eventuell sogar gesundheitsschädlichen Nebenwirkungen, die mit der Verwendung der vorbekannten Mittel in der Mesotherapie verbunden sind, vermieden werden.

Ferner ist es Aufgabe der Erfindung, ein Verfahren zu liefern, das eine lokale kosmetische Behandlung von Cellulitis ermöglicht.

Die Erfindung betrifft dementsprechend eine Zusammensetzung enthaltend
eine Komponente (a), die als Wirkstoff(e) 3,7-Dihydro-1,3-dimethyl-1*H*-purin-2,6-dion (Theophyllin) und/oder ein Theophyllinderivat enthält,
eine Komponente (b), die als Wirkstoff(e) 4-Chlor-2-furfurylamino-5-sulfamoylbenzoesäure (Furosemid) und/oder ein Derivat derselben enthält,
eine Komponente (c), die als Wirkstoff(e) eine Kombination aus 3',4',7-Tris-O-(2-hydroxyethyl) -rutin (Troxerutin) und 3-Hydroxy-1-methyl-5,6-indolindion-5-semicarbazon (Carbazochrom) und/oder eine Kombination von Derivaten derselben und/oder Roßkastanienextrakt enthält, und
eine Komponente (d), die als Wirkstoff(e) 2-Diethylamino-2',6'-dimethylacetanilid (Lidocain) und/oder p-Aminobenzoyldiethylaminoethanol (Procain) und/oder Derivate derselben enthält.

Gegenstand der Erfindung ist ferner die Verwendung einer derartigen Zusammensetzung als Arzneimittel oder kosmetisches Erzeugnis.

Gegenstand der Erfindung ist auch ein Verfahren zur kosmetischen Behandlung von Cellulitis, das dadurch gekennzeichnet ist, daß eine erfindungsgemäße Zusammensetzung mittels Mikroinjektionen im Bereich der betroffenen Hautbereiche intrakutan appliziert wird oder bei dem die Komponenten (a) bis (d) der erfindungsgemäßen Zusammensetzung mittels Mikroinjektionen voneinander getrennt im Bereich der betroffenen Hautbereiche intrakutan appliziert werden.

Bevorzugte Ausführungsformen der Erfindung ergeben sich aus den Unteransprüchen und der nachfolgenden Beschreibung.

Die erfindungsgemäße Zusammensetzung weist eine Komponente (a) auf, die als Wirkstoff(e) 3,7-Dihydro-1,3-dimethyl-1*H*-purin-2,6-dion (im folgenden: Theophyllin) und/oder ein Theophyllinderivat enthält. Theophyllin an sich und seine Herstellung sind im Stand der Technik bekannt. Erfindungsgemäß kann Theophyllin in der Komponente (a) zusammen mit geeigneten Lösungsvermittlern wie Monoethanolamin, Diethanolamin, Ethylendiamin und dergleichen verwendet werden. Auch können geeignete derivatisierte bzw. substituierte Theophylline verwendet werden, beispielsweise die durch Substituierung in 7-Stellung zugänglichen Derivate Hydroxyethyltheophyllin, Hydroxypropyltheophyllin, Dihydroxypropyltheophyllin. Beispielsweise kann Theophyllin in der Form der Molekülverbindung Aminophyllin, die aus 2 mol Theophyllin und 1 mol Ethylendiamin zusammengesetzt ist, verwendet werden. Vorzugsweise wird Theophyllin als Theophyllin-Ethylendiamin-Hydrat eingesetzt.

Komponente (b) der erfindungsgemäßen Zusammensetzung enthält als Wirkstoff(e) 4-Chlor-2-furfurylamino-5-sulfamoylbenzoesäure (im folgenden: Furosemid) und/oder ein geeignetes Derivat derselben. Furosemid an sich und seine Herstellung sind im Stand der Technik bekannt. Beispiele für geeignete derivatisierte Formen des Furosemids sind Benzoesäuresalze, zum Beispiel Alkalisalze. So wird Furosemid vorzugsweise in Form seines Natriumsalzes eingesetzt, das auch als Furosemid-Natrium bezeichnet wird.

Die Komponente (c) der erfindungsgemäßen Zusammensetzung enthält als Wirkstoff(e) eine Kombination aus dem Bioflavonoid 3',4',7-Tris-O-(2-hydroxyethyl)-rutin (nachfolgend: Troxerutin) und 3-Hydroxy-1-methyl-5,6-indolindion-5-semicarbazon (Carbazochrom) und/oder eine Kombination von Derivaten derselben und/oder Roßkastanienextrakt. Beispielsweise kann auch das Tris(hydroxymethyl)-Derivat des Rutins eingesetzt werden. Die genannten Wirkstoffe Troxerutin und Carbazochrom an sich sowie deren Herstellung sind im Stand der Technik bekannt. Ebenso ist die Gewinnung von Roßkastanienextrakt bekannt.

Roßkastanienextrakt wird häufig aus den Samen des Roßkastanienbaumes gewonnen. Inhaltsstoffe der Kastanienfrucht sind vorallem Triterpenglykoside (Aescin) und Flavone (Vitamin-P-Faktoren) sowie Adenin, Adenosin und cholinergische Stoffe. Aescin wird häufig nach speziellen Verfahren mit Hilfe von Ionenaustauschern gewonnen, wobei man unter Aescin die zu etwa 5 % in den getrockneten Samen der Roßkastanie enthaltene Saponinfraktion versteht.

Falls eine Kombination aus Troxerutin und Carbazochrom in Komponente (c) der erfindungsgemäßen Zusammensetzung verwendet wird, beträgt das Gewichtsverhältnis von Troxerutin zu Carbazochrom im allgemeinen 100 : 1 bis 2 : 1, vorzugsweise 50 : 1 bis 5 : 1 und insbesondere etwa 10 : 1.

Komponente (d) der erfindungsgemäßen Zusammensetzung enthält als Wirkstoff(e) 2-Diethylamino-2',6'-dimethylacetanilid (nachfolgend: Lidocain) und/oder p-Aminobenzoyldiethylaminoethanol (nachfolgend: Procain) und/oder Derivate derselben. Lidocain und Procain an sich sowie deren Herstellung sind im Stand der Technik bekannt. Die vorgenannten Wirkstoffe der Komponente (d) können vorteilhaft in Form der Hydrochloride eingesetzt werden. Vorzugsweise wird Lidocain- bzw. Procainhydrochlorid × 1 H₂O eingesetzt. Die Hydrochloride sind im allgemeinen durch die Einwirkung verdünnter Salzsäure auf Lidocain bzw. Procain zugänglich.

Weiterer Bestandteil der erfindungsgemäßen Zusammensetzungen bzw. der Komponenten (a) bis (d) ist im allgemeinen eine isotonische Kochsalzlösung, die für Injektionszwecke geeignet ist. Beispielsweise enthält sie NaCl, NaOH sowie Wasser für Injektionszwecke. Die Wirkstoffe der Komponenten (a) bis (d) sollten also in wasserlöslichen Formen vorliegen bzw. durch geeignete Derivatisierung in diese überführt werden.

Eine bevorzugte erfindungsgemäße Zusammensetzung enthält Theophyllin-Ethylendiamin-Hydrat, Furosemid-Natrium, eine Kombination aus Troxerutin und Carbazochrom, vorzugsweise im Verhältnis 10 : 1 (Gewichtsprozent), und Lidocain- und/oder Procain-Hydrochlorid.

Insbesondere liegen, bezogen auf die gesamte Zusammensetzung, der/die Wirkstoff(e) der Komponente (a) in einer Konzentration von 0,1 bis 20 mg/ml, vorzugsweise 1 bis 10 mg/ml und insbesondere 2 bis 9 mg/ml, der/die Wirkstoff(e) der Komponente (b) in einer Konzentration von 0,1 bis 20 mg/ml, vorzugsweise 0,1 bis 5 mg/ml und insbesondere 1 mg/ml, der/die Wirkstoff(e) der Komponente (c) in einer Konzentration von 0,1 bis 30 mg/ml, vorzugsweise 10 bis 20 mg/ml und insbesondere 15 bis 16 mg/ml und der/die Wirkstoff(e) der Komponente (d) in einer Konzentration von 0,1 bis 10 mg/ml, vorzugsweise 0,1 bis 5 mg/ml und insbesondere 1 mg/ml vor.

Bezieht man die Konzentrationsangaben jeweils auf die Konzentration des Wirkstoffs bzw. der Wirkstoffe in den einzelnen Komponenten (a) bis (d), so ergeben sich erfindungsgemäß geeignete Zusammensetzungen, wenn Komponente (a) als wäßrige Lösung mit einer Konzentration von 10 bis 40 mg/ml, vorzugsweise 20 bis 30 mg/ml und insbesondere 24 mg/ml, Komponente (b) als wäßrige Lösung mit einer Konzentration von 1 bis 30 mg/ml, vorzugsweise 5 bis 15 mg/ml und insbesondere 10 mg/ml, Komponente (c) als wäßrige Lösung mit einer Konzentration von 1 bis 200 mg/ml, vorzugsweise 1 bis 100 mg/ml und insbesondere 50 bis 51 mg/ml, und Komponente (d) als wäßrige Lösung mit einer Konzentration von 1 bis 100 mg/ml, vorzugsweise 1 bis 50 mg/ml und insbesondere 20 mg/ml verwendet wird.

Setzt man die Komponenten (a) bis (d) mit den im vorhergehenden Absatz genannten Wirkstoffkonzentrationen ein, erhält man eine erfindungsgemäß besonders geeignete eine Zusammensetzung, wenn bezogen auf 10 ml Gesamtvolumen
0,1 bis 5 ml, vorzugsweise 0,5 bis 4 ml und insbesondere 1 bis 3,5 ml der Komponente (a),
0,1 bis 5 ml, vorzugsweise 0,5 bis 3 ml und insbesondere 1 ml der Komponente (b),
0,1 bis 5 ml, vorzugsweise 2 bis 4 ml und insbesondere 3 ml der Komponente (c), und
0,1 bis 5 ml, vorzugsweise 0,1 bis 2 ml und insbesondere 0,5 ml der Komponente (d)
zusammengegeben werden. Der Rest der Zusammensetzung (Differenz zu 10 ml Gesamtvolumen) besteht im wesentlichen aus einer wäßrigen isotonischen Natriumchloridlösung.

Die oben beschriebenen erfindungsgemäßen Zusammensetzungen werden im allgemeinen einfach durch Zusammengeben bzw. Vermischen der gelösten Wirkstoffe hergestellt. Es können auch erst die festen/flüssigen Wirkstoffe miteinander vermischt werden und dann gemeinsam mit einem geeigneten Lösungsmittel gelöst werden. Dabei müssen die Lösungsmittel für Injektionszwecke geeignet sein. Neben den eigentlichen Wirkstoffen können also noch Natriumchlorid, Natriumhydroxid und dergleichen sowie Wasser für Injektionszwecke zugegeben werden.

Die erfindungsgemäßen Zusammensetzungen können als Arzneimittel oder kosmetisches Erzeugnis verwendet werden.

Insbesondere können die erfindungsgemäßen Zusammensetzungen zur kosmetischen Behandlung von Cellulitis eingesetzt werden. Diese Behandlung erfolgt in der Regel derart, daß die oben beschriebene Zusammensetzung mittels Mikroinjektionen im Bereich der von Cellulitis betroffenen Hautbereiche intrakutan, d.h. in die sogenannte Lederhaut, appliziert wird.

Zur Mikroinjektion werden beispielsweise Nadeln mit einer Länge von 4 mm verwendet. Derartige Injektionsnadeln sind aus der Mesotherapie bekannt, worunter im allgemeinen die Verabreichung von niedrig dosierten Substanzen durch Mikroinjektionen in die Lederhaut verstanden wird. Diese kann mit Hilfe sogenannter Multiinjektoren oder einzelner Kanülen erfolgen. Ein Multiinjektor ist dadurch gekennzeichnet, daß er mehrere in einem bestimmten Abstand voneinander angeordnete Kanülen aufweist und so die gleichzeitige Injektion über einen räumlich ausgedehnten Hautbereich erlaubt. Beispielsweise besteht der Multiinjektor aus einem Gefäß, das auf der einen Seite eine Öffnung für ein Vorratsgefäß zur Aufnahme der erfindungsgemäßen Zusammensetzung aufweist, und auf der anderen Seiten drei, vier oder fünf nebeneinander angeordnete Öffnungen zur Aufnahme von Kanülen für Mikroinjektionen.

Es ist ebenfalls möglich, die Komponenten (a) bis (d) der oben beschriebenen Zusammensetzung mittels einzelner Mikroinjektionen kurz hintereinander und somit voneinander getrennt, jedoch im Bereich der betroffenen Hautbereiche, intrakutan zu verabreichen. Ebenfalls kann man nur einen Teil der Komponenten (a) bis (d) zusammen und die restlichen Komponenten getrennt injizieren. Beispielsweise kann man also Komponenten (a) und (b) zusammen und danach (c) und (d) getrennt injizieren. Oder (a) und (c) werden zusammen und (b) und (d) getrennt verabreicht. Besonders vorteilhaft ist jedoch die Verabreichung der erfindungsgemäßen Zusammensetzung als Mischung der Komponenten (a) bis (d) mit Hilfe eines Multiinjektors. Dabei wird die synergistische Wirkung der erfindungsgemäßen Zusammensetzung besonders deutlich.

Wie schon beschrieben, erfolgt die Verabreichung der erfindungsgemäßen Zusammensetzung in die von Cellulitis betroffenen Hautbereiche, d.h. im allgemeinen in die Außen- und Innenseite des Oberschenkels. Die Behandlung erfolgt im allgemeinen 1- bis 2mal pro Woche über einen Zeitraum von 2 Wochen, danach 1mal innerhalb von 2 Wochen für 2 Monate. Als Erhaltungsdosis ist im allgemeinen eine Injektion pro Monat erforderlich. Je nach Grad der Cellulitis, kann jedoch von diesem Anwendungsschema abgewichen werden. Pro Behandlung wird im allgemeinen eine Gesamtdosis von 10 ml der erfindungsgemäßen Zusammensetzung mit den oben beschriebenen Konzentrationen der Wirkstoffe verabreicht.

Das erfindungsgemäße Verfahren weist den Vorteil auf, daß durch die lokale Anwendung in den betroffenen Hautbereichen und durch die niedrigen Konzentrationen der Wirkstoffe in den Komponenten (a) bis (d) geringere Nebenwirkungen zu erwarten sind als bei oralen oder intramuskulären Applikationen.

### Ausführungsbeispiele

### Beispiel 1

Eine erfindungsgemäße Zusammensetzung wurde hergestellt, indem 1 ml einer Lösung von Theophyllin-Ethylendiamin-Hydrat mit einer Konzentration von 24 mg/ml in Wasser für Injektionszwecke (weitere Bestandteile Ethylendiamin), 1 ml einer Lösung von Furosemid-Natrium mit einer Konzentration von 10,7 mg/ml in Wasser für Injektionszwecke (weitere Bestandteile Natriumchlorid, Natriumhydroxid), 3 ml einer Lösung von Troxerutin und Carbazochrom mit einer Konzentration von 50 mg/l Troxerutin und 0,5 mg/ml Carbazochrom in Wasser für Injektionszwecke (weitere Bestandteile Natriumchlorid, Natriumhydroxid) und 0,5 ml einer Lösung von Lidocainhydrochlorid 1 H₂O mit einer Konzentration von 20 mg/ml in Wasser für Injektionszwecke (weitere Bestandteile Natriumchlorid, Natriumhydroxid) und 4,5 ml isotonische Natriumchloridlösung zusammengegeben wurden.

### Beispiel 2

Eine erfindungsgemäße Zusammensetzung wurde wie in Beispiel 1 hergestellt, mit der Ausnahme, daß anstelle von 1 ml einer Lösung von Theophyllin-Ethylendiamin-Hydrat mit einer Konzentration von 24 mg/ml in Wasser für Injektionszwecke (weitere Bestandteile Ethylendiamin) 3,5 ml dieser Lösung und 2 ml isotonische Natriumchloridlösung verwendet wurden.

### Beispiel 3

Eine Testperson mit Cellulitis wurde behandelt, indem mittels eines Mikroinjektors (3 Kanülen von 4 mm Länge) eine Dosis von 10 ml der Zusammensetzung aus Beispiel 1 ein- bis zweimal pro Woche für 2 Wochen, danach 1mal alle 14 Tage für 2 Monate unmittelbar in den von Cellulitis betroffenen Hautbereichen injiziert wurde. Nach dieser Zeit waren die behandelten Hautbereiche deutlich glatter geworden und erschienen erheblich gestrafft. Das typische symptomatische Bild durch Cellulitis war deutlich zurückgetreten.

Entsprechende Behandlungen mit einer großen Zahl weiterer Personen ergab ähnliche Ergebnisse, das heißt die behandelten Personen waren nach der Behandlung cellulitefrei oder es trat zumindest eine deutliche Reduzierung der Cellulitis ein.

## Patentansprüche

1. Zusammensetzung, enthaltend
eine Komponente (a), die als Wirkstoff(e) 3,7-Dihydro-1,3-dimethyl-1H-purin-2,6-dion (Theophyllin) und/oder ein Theophyllinderivat enthält,
eine Komponente (b), die als Wirkstoff(e) 4-Chlor-2-furfurylamino-5-sulfamoylbenzoesäure (Furosemid) und/oder ein Derivat derselben enthält,
eine Komponente (c), die als Wirkstoff(e) eine Kombination aus 3',4',7-Tris-O-(2-hydroxyethyl) -rutin (Troxerutin) und 3-Hydroxy-1-methyl-5,6-indolindion-5-semicarbazon (Carbazochrom) und/oder eine Kombination von Derivaten derselben und/oder Roßkastanienextrakt enthält, und
eine Komponente (d), die als Wirkstoff(e) 2-Diethylamino-2',6'-dimethylacetanilid (Lidocain) und/oder p-Aminobenzoyldiethylaminoethanol (Procain) und/oder Derivate derselben enthält.

2. Zusammensetzung nach Anspruch 1, **dadurch gekennzeichnet, daß** die Komponente (a) Theophyllin-Ethylendiamin-Hydrat, die Komponente (b) Furosemid-Natrium, die Komponente (c) eine Kombination aus Troxerutin und Carbazochrom und die Komponente (d) Lidocain- und/oder Procainhydrochlorid enthält.

3. Zusammensetzung nach Anspruch 1 oder 2, **dadurch gekennzeichnet, daß** bezogen auf die gesamte Zusammensetzung die Wirkstoffe der Komponenten
(a) in einer Konzentration von 0,1 bis 20 mg/ml, vorzugsweise 1 bis 10 mg/ml und insbesondere 2 bis 9 mg/ml,
(b) in einer Konzentration von 0,1 bis 20 mg/ml, vorzugsweise 0,1 bis 5 mg/ml und insbesondere 1 mg/ml,
(c) in einer Konzentration von 0,1 bis 30 mg/ml, vorzugsweise 10 bis 20 mg/ml und insbesondere 15 bis 16 mg/ml und
(d) in einer Konzentration von 0,1 bis 10 mg/ml, vorzugsweise 0,1 bis 5 mg/ml und insbesondere 1 mg/ml
vorliegen.

4. Zusammensetzung nach Anspruch 1 oder 2, **dadurch gekennzeichnet, daß** die Komponenten
(a) als wäßrige Lösung mit einer Konzentration von 10 bis 40 mg/ml, vorzugsweise 20 bis 30 mg/ml und insbesondere 24 mg/ml,
(b) als wäßrige Lösung mit einer Konzentration von 1 bis 30 mg/ml, vorzugsweise 5 bis 15 mg/ml und insbesondere 10 mg/ml,
(c) als wäßrige Lösung mit einer Konzentration von 1 bis 200 mg/ml, vorzugsweise 1 bis 100 mg/ml und insbesondere 50 bis 51 mg/ml, und
(d) als wäßrige Lösung mit einer Konzentration von 1 bis 100 mg/ml, vorzugsweise 1 bis 50 mg/ml und insbesondere 20 mg/ml
vorliegen, wobei sich die Konzentrationsangaben jeweils auf die Konzentration des Wirkstoffs bzw. der Wirkstoffe in den einzelnen Komponenten (a) bis (d) beziehen.

5. Zusammensetzung nach Anspruch 4, **dadurch gekennzeichnet, daß** sie bezogen auf 10 ml Gesamtvolumen
0,1 bis 5 ml, vorzugsweise 0,5 bis 4 ml und insbesondere 1 bis 3,5 ml der Komponente (a),
0,1 bis 5 ml, vorzugsweise 0,5 bis 3 ml und insbesondere 1 ml der Komponente (b),
0,1 bis 5 ml, vorzugsweise 2 bis 4 ml und insbesondere 3 ml der Komponente (c), und
0,1 bis 5 ml, vorzugsweise 0,1 bis 2 ml und insbesondere 0,5 ml der Komponente (d)
enthält, wobei der Rest der Zusammensetzung im wesentlichen aus einer wäßrigen isotonischen Natriumchloridlösung besteht.

6. Verwendung einer Zusammensetzung gemäß einem der Ansprüche 1 bis 5 als Kosmetikum.

7. Verwendung einer Zusammensetzung gemäß den Ansprüchen 1 bis 5 zur Herstellung eines Arzneimittels.

8. Verfahren zur kosmetischen Behandlung von Cellulitis, **dadurch gekennzeichnet, daß** eine Zusammensetzung gemäß einem der Ansprüche 1 bis 5 mittels Mikroinjektionen im Bereich der betroffenen Hautbereiche intrakutan appliziert wird.

9. Verfahren zur kosmetischen Behandlung von Cellulitis, **dadurch gekennzeichnet, daß** die Komponenten (a) bis (d) der Zusammensetzung gemäß einem der Ansprüche 1 bis 5 mittels Mikroinjektionen voneinander getrennt oder teilweise kombiniert im Bereich der betroffenen Hautgebiete intrakutan appliziert werden.

10. Verfahren nach Anspruch 8 oder Anspruch 9, **dadurch gekennzeichnet, daß** die Injektionen zuerst ein- bis zweimal pro Woche für 2 Wochen, dann 1mal alle 2 Wochen für 2 Monate und anschließend 1 mal pro Monat erfolgen.

11. Verfahren nach Anspruch 8, **dadurch gekennzeichnet, daß** die Komponenten (a) bis (d) getrennt aufbewahrt werden und unmittelbar vor der Injektion zu einer Zusammensetzung zusammengegeben werden.

## Claims

1. Composition containing
a component (a) containing as active substance(s) 3,7-dihydro-1,3-dimethyl-1*H*-purine-2,6-dione (theophylline) and/or a theophylline derivative;
a component (b) containing as active substance(s) 4-chloro-2-furfurylamino-5-sulfamoylbenzoic acid (furosemide) and/or a derivative thereof;
a component (c) containing as active substance(s) a combination of 3',4',7-tris-O-(2-hydroxyethyl)-rutin (troxerutin) and 3-hydroxy-1-methyl-5,6-indolinedione-5-semicarbazone (carbazochrome) and/or a combination of derivatives thereof and/or horse chestnut extract ; and
a component (d), containing as active substance(s) 2-diethylamino-2',6'-dimethylacetanilide (lidocaine) and/or p-aminobenzoyl diethylaminoethanol (procaine) and/or derivatives thereof.

2. Composition according to claim 1, **characterised in that** said component (a) contains theophylline ethylenediamine hydrate, said component (b) contains furosemide sodium, said component (c) contains a combination of troxerutin and carbazochrome, and said component (d) contains lidocaine and/or procaine hydrochloride.

3. Composition according to claim 1 or 2, **characterised in that**, based on the total composition, the active substances of
component (a) are present in a concentration of 0,1 to 20 mg/ml, preferably 1 to 10 mg/ml and in particular 2 bis 9 mg/ml;
component (b) are present in a concentration von 0,1 to 20 mg/ml, preferably 0,1 to 5 mg/ml and in particular 1 mg/ml;
component (c) are present in a concentration von 0,1 to 30 mg/ml, preferably 10 to 20 mg/ml and in particular 15 to 16 mg/ml and
component (d) are present in a concentration von 0,1 to 10 mg/ml, preferably 0,1 to 5 mg/ml and in particular 1 mg/ml .

4. Composition according to claim 1 oder 2, **characterised in that**
component (a) is present in form of an aqueous solution having a concentration of 10 to 40 mg/ml, preferably 20 to 30 mg/ml and in particular 24 mg/ml;
component (b) is present in form of an aqueous solution having a concentration von 1 to 30 mg/ml, preferably 5 to 15 mg/ml and in particular 10 mg/ml;
component (c) is present in form of an aqueous solution having a concentration von 1 to 200 mg/ml, preferably 1 to 100 mg/ml and in particular 50 to 51 mg/ml; and
component (d) is present in form of an aqueous solution having a concentration of 1 to 100 mg/ml, preferably 1 to 50 mg/ml and in particular 20 mg/ml,
wherein the said concentrations are relative to the active substance concentration(s) in the individual components (a) to (d).

5. Composition according to claim 4, **characterised in that** it contains, on the basis of a total volume of 10 ml,
0,1 to 5 ml, preferably 0,5 to 4 ml and in particular 1 to 3,5 ml of component (a);
0,1 to 5 ml, preferably 0,5 to 3 ml and in particular 1 ml of component (b);
0,1 to 5 ml, preferably 2 to 4 ml and in particular 3 ml of component (c); and
0,1 to 5 ml, preferably 0,1 to 2 ml and in particular 0,5 ml of component (d),
wherein the balance of the composition consists essentially of an aqueous isotonic sodium chloride solution.

6. Use of a composition according to any of claims 1 to 5 as a cosmetic.

7. Use of a composition according to any of claims 1 to 5 for the manufacture of a medicament.

8. Method for cosmetic treatment of cellulitis, **characterised in that** a composition according to any of claims 1 to 5 is administered intracutaneaously by means of micro injections to the area of the affected areas of the skin.

9. Method for cosmetic treatment of cellulitis, **characterised in that** the components (a) to (d) of the composition according to any of claims 1 to 5 are administered intracutaneaously separately or partially combined, by means of micro injections, to the area of the affected areas of the skin.

10. Method according to claim 8 oder claim 9, **characterised in that** the injections are performed once to twice per week for 2 weeks, then once every 2 weeks for 2 months, and subsequently once per month.

11. Method according to claim 8, **characterised in that** said components (a) to (d) are stored separately, and are combined into a composition immediately prior to the injection.

## Revendications

1. Composition contenant
un constituant (a) contenant comme substance(s) active(s) de la 3,7-dihydro-1,3-diméthyl-1H-purine-2,6-dione (théophylline) et/ou un dérivé de théophylline,
un constituant (b) contenant comme substance(s) active(s) de l'acide 4-chloro-2-furfurylamino-5-sulfamoylbenzoïque (furosémide) et/ou un de ses dérivés,
un constituant (c), contenant comme substance(s) active(s) une combinaison de 3',4',7-tris-O-(2-hydroxyéthyl)rutine (troxérutine) et de 3-hydroxy-1-méthyl-5,6-indolinedione-5-semicarbazone (carbazochrome) et/ou une combinaison de dérivés de ces substances et/ou un extrait de marron d'Inde, et
un constituant (d) contenant comme substance(s) active(s) du 2-diéthylamino-2',6'-diméthylacétanilide (lidocaïne) et/ou du p-aminobenzoyldiéthylaminoéthanol (procaïne) et/ou des dérivés de ces substances.

2. Composition selon la revendication 1, **caractérisée en ce que** le constituant (a) contient de la théophylline-éthylènediamine hydratée, le constituant (b) du furosémide-sodium, le constituant (c) une combinaison de troxérutine et de carbazochrome et le constituant (d) du chlorhydrate de lidocaïne et/ou de procaïne.

3. Composition selon la revendication 1 ou 2, **caractérisée en ce que**, par rapport à la composition totale,
les substances actives du constituant (a) sont présentes à une concentration de 0,1 à 20 mg/l, de préférence de 1 à 10 mg/l et en particulier de 2 à 9 mg/l,
les substances actives du constituant (b) sont présentes à une concentration de 0,1 à 20 mg/l, de préférence de 0,1 à 5 mg/l et en particulier de 1 mg/l,
les substances actives du constituant (c) sont présentes à une concentration de 0,1 à 30 mg/l, de préférence de 10 à 20 mg/l et en particulier de 15 à 16 mg/l, et
les substances actives du constituant (d) sont présentes à une concentration de 0,1 à 10 mg/l, de préférence de 0,1 à 5 mg/l et en particulier de 1 mg/l.

4. Composition selon la revendication 1 ou 2, **caractérisée en ce que**
le constituant (a) est sous forme d'une solution aqueuse à une concentration de 10 à 40 mg/ml, de préférence de 20 à 30 mg/ml et en particulier de 24 mg/ml,
le constituant (b) est sous forme d'une solution aqueuse à une concentration de 1 à 30 mg/ml, de préférence de 5 à 15 mg/ml et en particulier de 10 mg/ml,
le constituant (c) est sous forme d'une solution aqueuse à une concentration de 1 à 200 mg/ml, de préférence de 1 à 100 mg/ml et en particulier de 50 à 51 mg/ml, et
le constituant (d) est sous forme d'une solution aqueuse à une concentration de 1 à 100 mg/ml, de préférence de 1 à 50 mg/ml et en particulier de 20 mg/ml,
les indications de concentration se rapportant toujours à la concentration de la substance active ou des substances actives dans les différents constituants (a) à (d).

5. Composition selon la revendication 4, **caractérisée en ce qu'**elle contient, pour 10 ml de volume total
0,1 à 5 ml, de préférence 0,5 à 4 ml et en particulier 1 à 3,5 ml de constituant (a),
0,1 à 5 ml, de préférence 0,5 à 3 ml et en particulier 1 ml de constituant (b),
0,1 à 5 ml, de préférence 2 à 4 ml et en particulier 3 ml de constituant (c), et
0,1 à 5 ml, de préférence 0,1 à 2 ml et en particulier 0,5 ml de constituant (d),
le reste de la composition étant essentiellement constitué d'une solution aqueuse isotonique de chlorure de sodium.

6. Utilisation d'une composition selon l'une des revendications 1 à 5 comme composition cosmétique.

7. Utilisation d'une composition selon les revendications 1 à 5 pour la préparation d'un médicament.

8. Procédé de traitement cosmétique de la cellulite, **caractérisé en ce que** l'on applique par voie intracutanée une composition selon l'une des revendications 1 à 5 à l'aide de micro-injections dans la zone des régions de peau touchées.

9. Procédé de traitement cosmétique de la cellulite, **caractérisé en ce que** l'on applique par voie intracutanée les constituants (a) à (d) de la composition selon l'une des revendications 1 à 5, séparément les uns des autres ou de façon partiellement combinée, à l'aide de micro-injections dans la zone des régions de peau touchées.

10. Procédé selon la revendication 8 ou la revendication 9, **caractérisé en ce que** les injections s'effectuent d'abord une à deux fois par semaine pendant 2 semaines, puis 1 fois toutes les 2 semaines pendant 2 mois, et ensuite 1 fois par mois.

11. Procédé selon la revendication 8, **caractérisé en ce que** l'on conserve séparément les constituants (a) à (d) et **en ce qu'**on les mélange en une composition juste avant l'injection.
